# EUROPEAN PATENT APPLICATION

(11) **EP 4 800 701 A1**
(43) Date of publication of application: **02.09.2026**
(21) Application number: 25160981.4
(22) Date of filing: 28.02.2025
(51) Int. Cl.: G16H 30/40, G06T 7/11, G06T 19/20, A61B 34/10

(54) **REAL-TIME DYNAMIC ORGAN MODELLING AND IMAGE AUGMENTATION IN SURGERY**

(71) Applicant: Orsi Academy bv, 9090 Melle (BE)
(72) Inventor: MOTTRIE, Alexandre, 9090 Melle (BE); HOFMAN, Jasper, 9090 Melle (BE); DE BACKER, Pieter, 9090 Melle (BE)
(74) Representative: Brantsandpatents bv

(57) **Abstract**

The current invention relates to a method for real-time organ model modelling based on medical image data, wherein the computational requirements are reduced. In some aspects, these organ models can be used to augment the medical images in real-time.

## Description

### FIELD OF THE INVENTION

The present invention relates to a method for real-time organ modelling and potential real-time image augmentation during medical procedures, such as surgery.

### BACKGROUND

In the current state of the art, more and more use is made of artificial intelligence to provide real-time tracking and augmentation of (medical) images, that can assist medical practitioners during procedures, but can also be used afterwards for analysis.

One of the main issues here is that they are often computationally complex, thus creating a lag, while the medical practitioner needs essentially real-time feedback. This is not only because there is a strong urgency for this feedback in order to avoid errors or accidents, and be able to react as soon as possible, or even prevent aggravating the situation (for instance, cutting further at an incorrect location). Another reason for the need to get this information as soon as possible, is that the medical practitioner may rely on this for guidance in the procedure, and needs immediate and accurate images and information. Having lag or delay between what they actually do and what they perceive in images or in terms of received information creates a separation between the motor system and the senses that is confusing, increases complexity of the task and requires intense concentration and focus while other things require the attention of the medical practitioner.

In essence, reducing lag to an absolute minimum is absolutely crucial. At present, this is not achievable without excessively large computational capacities.

The invention aims to resolve this issue, by simplifying the computational complexity of the tracking process.

### SUMMARY OF THE INVENTION

In a first aspect, the invention relates to a computer-implemented method for real-time organ detection in medical procedures on a patient, comprising the steps of:
a. receiving a plurality of digital images of a medical procedure, the plurality of digital images being a video;
b. automatically segmenting the digital images by a pretrained machine learning model into image segments and separating and identifying the segmented image segments in two or more classes, said classes comprising at least a class of organ tissue;
c. preparing one or more three-dimensional organ models based on historical image data of said patient;
d. based on the image segments identified as organ tissue, detecting at least one organ in a first digital image of the plurality of digital images;
e. adapting the prepared organ model of the detected organ for the first digital image, wherein the adaptation of said organ model is performed based on the identified organ tissue of the detected organ in said first digital image such that the organ model substantially corresponds to the detected organ in the first digital image in size and orientation, and optionally position;
f. determining in at least one subsequent digital image to the first digital image, a rotation and/or resizing, and/or an optional translation of the identified organ tissue of the organ with respect to the identified organ tissue of the organ in the first digital image, and adapting the three-dimensional organ for the subsequent digital image by transforming the organ model for the first image according to said determined rotation and/or resizing and/or optional translation;
g. determining in at least one further subsequent digital image to the first digital image, preferably said further subsequent digital image being subsequent to the subsequent digital image, a further rotation and/or resizing and/or an optional translation of the identified organ tissue of the organ with respect to the identified organ tissue of the organ in a preceding digital image for which a three-dimensional organ model was adapted, and adapting the three-dimensional organ model for the further subsequent digital image by transforming the organ model for the preceding digital image according to said determined further rotation and/or resizing and/or optional translation.

In a further aspect, the invention relates to the method being used for real-time image augmentation during medical procedures, specifically during surgery, wherein the digital images are displayed and wherein the organ models are provided as an organ model overlay over the digital images.

In a further aspect, the invention relates to the use of the methods of the invention for post-procedure analysis, in terms of procedure quality control, detection of potential issues that require postoperative follow-up and were missed or could not be followed up during the procedure itself, and others.

In another further aspect, the invention relates to the use of the methods of the invention for real-time image augmentation during the medical procedure, by providing the organ models as an overlay over the digital images of the procedure.

### DESCRIPTION OF FIGURES

**Figures 1A****-B-C-D** shows subsequent stills from a video comprising a plurality of digital images that were processed according to an embodiment of the method of the invention.
**Figure 2** shows a digital image that was provided with an organ overlay and a non-organic (tool) overlay in steps.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In a first aspect, the invention relates to a computer-implemented method for real-time organ detection in medical procedures on a patient, comprising the steps of:
a. receiving a plurality of digital images of a medical procedure, the plurality of digital images being a video;
b. automatically segmenting the digital images by a pretrained machine learning model into image segments and separating and identifying the segmented image segments in two or more classes, said classes comprising at least a class of organ tissue;
c. preparing one or more three-dimensional organ models based on historical image data of said patient;
d. based on the image segments identified as organ tissue, detecting at least one organ in a first digital image of the plurality of digital images;
e. adapting the prepared organ model of the detected organ for the first digital image, wherein the adaptation of said organ model is performed based on the identified organ tissue of the detected organ in said first digital image such that the organ model substantially corresponds to the detected organ in the first digital image in size and orientation, and optionally position;
f. determining in at least one subsequent digital image to the first digital image, a rotation and/or resizing, and/or an optional translation of the identified organ tissue of the organ with respect to the identified organ tissue of the organ in the first digital image, and adapting the three-dimensional organ for the subsequent digital image by transforming the organ model for the first image according to said determined rotation and/or resizing and/or optional translation;
g. determining in at least one further subsequent digital image to the first digital image, preferably said further subsequent digital image being subsequent to the subsequent digital image, a further rotation and/or resizing and/or an optional translation of the identified organ tissue of the organ with respect to the identified organ tissue of the organ in a preceding digital image for which a three-dimensional organ model was adapted, and adapting the three-dimensional organ model for the further subsequent digital image by transforming the organ model for the preceding digital image according to said determined further rotation and/or resizing and/or optional translation.

In a variation, the method can even be reduced to the following steps if an organ model is already provided for a first digital image such that it matches the organ in said first digital image:
a. receiving a plurality of digital images of a medical procedure, the plurality of digital images being a video;
b. automatically segmenting the digital images by a pretrained machine learning model into image segments and separating and identifying the segmented image segments in two or more classes, said classes comprising at least a class of organ tissue;
c. determining in at least one subsequent digital image to the first digital image, a rotation and/or resizing, and/or an optional translation of the identified organ tissue of the organ with respect to the identified organ tissue of the organ in the first digital image, and adapting the three-dimensional organ for the subsequent digital image by transforming the organ model for the first image according to said determined rotation and/or resizing and/or optional translation;
d. determining in at least one further subsequent digital image to the first digital image, preferably said further subsequent digital image being subsequent to the subsequent digital image, a further rotation and/or resizing and/or an optional translation of the identified organ tissue of the organ with respect to the identified organ tissue of the organ in a preceding digital image for which a three-dimensional organ model was adapted, and adapting the three-dimensional organ model for the further subsequent digital image by transforming the organ model for the preceding digital image according to said determined further rotation and/or resizing and/or optional translation.

Throughout the application, on the one side a coupling will be made of the first digital image and the subsequent digital image, and on the other side a coupling of the preceding digital image and the further subsequent digital image. In essence, these couplings are arbitrary in all ways excepting that the organ model for the first digital image was generated or adapted directly. However, when discussing the above couplings, this does not substantially impact the steps, features, or techniques used.

For brevity, the description will often describe certain steps, a scenario or example referring to the further subsequent digital images and the preceding image, but what is described will be applicable to the coupling of the subsequent digital image and the first digital image as well, unless the contrary is explicitly stated.

In known methods for organ detection/modelling in medical procedures, the bottleneck lies in the generation of the organ model(s) for each image separately, where the image is usually segmented, and then processed to map an organ model onto the organ in the images. By doing this separately for each image, the computational load is extremely high, which creates a lag in most cases. This is of course suboptimal, as the lag creates both a delay in the potential reaction of a medical practitioner to what is show, but also, and very crucially in surgery for instance, creates a dissociation between the motor system of the practitioner and what they are receiving as (visual) input due to the lag between the two.

Instead of preparing the organ model for each image separately by mapping it onto the segmented image, the current invention tries to avoid this as much as possible. Instead, it performs this action a first time for a first digital image, generating the organ model for this first digital image specifically by adapting the original organ model to this first digital image, by positioning it in the correct location, by changing the size to match that in the digital image and by adjusting the orientation of the (three-dimensional) organ model such that it matches that in the digital image. This step can be performed in an automated fashion, for instance via an iterative closest point (ICP) algorithm, or via a neural network,

This is often a difficult step in terms of computational capacity that is required, and is not easily implementable for all images, because the organ is insufficiently visible to adapt the organ model based solely on the images, but also because this step can induce (excessive) lag.

In some variations, this initial adaptation step is done manually, by an operator aligning the organ model with the first digital image.

The original organ model was generated based on historical medical images for the patient, in order to have a correct and representative organ model, which again reduces the computational load required.

For the subsequent and the further subsequent digital images however, the method will not re-perform the above actions for the first digital image. Instead, it will use the adapted organ model of a preceding image (the first digital image or another) and apply a transformation onto it such that it matches the organ's position/orientation/size in the (further) subsequent digital images.

This transformation is determined based on a comparison of the segmented digital images of the further subsequent and the preceding digital images (or for the subsequent and first digital images). The comparison allows a transformation to be determined of the organ (tissue) between the two digital images, and this transformation to then be applied to the organ model of the preceding digital image, which is computationally less constraining than remapping the organ model from scratch for each digital image. In turn, this reduces the lag and thus increases the synchronicity of movement in view of the actions of the practitioner.

Additionally, if the organ model, as in the prior art, is adapted independently for each digital image, there is a high risk of error, due to insufficiently clear images (partial occlusion of the organ, lens stains). Instead, a deliberate choice is made to depart from a first highly accurate mapping in the first image, and using landmarks to 'orient' the new position, orientation and size of the organ in (further) subsequent images.

What is required, is therefore a starting point, namely an image in which the organ is mapped substantially correctly. Because of this, even more care can be taken for this initial mapping to be very accurate, since this will in turn increase the correctness of the organ model for future digital images. Even more handily, in the initial digital images, often there is no interference yet of tools or other foreign objects (gauze, etc.) that can occlude the visibility of the organ, which will further increase the accuracy. In known methodologies, during many moments in the procedure, the visibility of the organs will be severely limited and will substantially add to the complexity and the computational load, while in the present methodology, this computational is essentially stacked at the beginning (at simpler images), and is reduced at the later stages.

In some embodiments, the method will undergo periodical checks in terms of performing a (parallel or postponed) check of the organ model being used versus an organ model being mapped as in step e for a digital image at a later stage. If the mismatch is greater than a predetermined amount, the method can choose to reset and return to step e and then continue again with steps f and g. This allows a periodical check and, if necessary, correction in case the transformations of step f and g have caused a divergence with respect to the correct position. As mentioned, this can be done in parallel, but does not need to be performed real-time. The check can be done at a more leisurely rate (although this will not be more than a few seconds in most cases), which will allow the method to still correct itself quick enough.

The predetermined amount of mismatch can be set by the practitioner, but will take into account the position, orientation and size of the organ model according to step g and compare it to an organ model prepared according to step e for a further subsequent digital image.

In some cases, or additionally to the above embodiment, the method can further be configured to "reset" at fixed times, such as every minute, every 2 minutes, every 5 minutes, etc.

In some embodiments, the organ model is only transformed to represent a correct size and orientation. In some embodiments, even the size can omitted, and only the orientation is changed. This is because the practitioner can in most occasions easily deduce these parameters for the organ, while the orientation is sometimes more difficult to deduce.

However, optimally, the organ model is adapted to represent both the correct size, orientation and positioning in the digital image, allowing it to be used for further analysis, for creating overlays, for detection of interaction between the organ and tools, other organs, or other objects, etc.

Of course, it is clear that the adaptation will in some cases have no rotation, translation or resizing if there is no or a minute difference between the two images (further subsequent and preceding image, or subsequent and first image). In such cases, the organ model will remain the same for the two images.

In some embodiments, the organ model will only be adapted periodically instead of for every digital image, which further reduces computational load. This can be a static pattern, for instance once every X images, with X being 2, 3, 4, 5, 6, 8, 10, 12, 18, 24, 32, 64, 128, etc., images, depending on the frame rate. This can also be a dynamic pattern, where the method can differentiate between more static (small changes to the images) and more dynamic (high amount of changes between images) image sequences. In case of more static image sequences, the adaptation can be performed less frequently, skipping a higher amount of images, while in more dynamic situations, the organ model is adapted more frequently. This can be according to a fixed model with two or more "levels" in terms of dynamic nature of the image sequence, with the levels have a specific adaptation frequency, but this can also be a more fluent system (for instance, formulaic based on the dynamic nature).

In a preferred embodiment, when an organ is no longer (sufficiently) visible in a digital image, a number of options can be used, which can be used separately or as fallbacks, depending also on the situation. If the organ is not (sufficiently) visible, typically no organ model will be adapted and associated to that digital image. When the organ becomes visible again in later digital images that would use the digital image with no associated organ model to determine the transformation, the following options exist:
- reverting to earlier preceding digital images, preceding the digital image with no associated organ model. Preferably, these earlier preceding digital images are directly preceding (i.e., the digital image with the latest timestamp before the image with no associated organ model). If the closest earlier preceding digital image also has no associated organ model, then the digital image before that is taken until a digital image is found that does have an associated organ model;
- repeating step e to adapt the original organ model to the lateral digital image in which the organ is (sufficiently) visible again, thus essentially restarting the method.

The possibility for combination of the above options in cascading order can for instance be based on the length of time over which no earlier preceding digital images can be found that have an associated organ model, or based on a threshold difference "value" between the image in which the organ is visible again and preceding digital images over a certain span of time, meaning that if these differ too strongly, the organ model is adapted according to step g, and if the difference is below the threshold difference value, then the organ is adapted based on the earlier organ model.

In some embodiments, an organ model can still be provided for the images where the organ is not (sufficiently) visible in a closest fit, or by reusing the previous organ model. Preferably, this is however classified as not suitable for the further steps of adapting the further organ models for later images.

In a preferred embodiment, the classes further comprise a class of non-organic objects. Optionally, the classes further comprise a class of background tissue (i.e., not non-organic objects and not organ tissue).

This allows the method to also track instruments and other foreign objects, which can be useful to determine tool-organ interaction or proximity, which can be used to warn or alert the practitioner. It furthermore can be used to keep track of objects to ensure later removal (gauze, etc.), and generally, to further differentiate from the other types of tissue present. Furthermore, by classifying background tissue separately, the differentiating for organ tissue (and tools) is optimized further.

In a preferred embodiment, the class of organ tissue is subdivided in subclasses per organ. This allows differentiation between separate organs, and allowing tracking of the separate organs via separate organ models. The list of organs that are considered as separate subclasses need not be complete, and may comprise one or more of: kidneys, colon, peritoneum, fat tissue, diaphragm, liver, spleen, artery, ureter, ligament, vessel, muscle, vein, duodenum, small intestine, large intestine, gallbladder, stomach, heart, lungs,

In a further preferred embodiment, the organs are further supplemented by one or more specific tissue masses that technically do not qualify as organs, such as tumors, fat tissue, cysts, and other types of neoplasm etc. Specifically, the tumor and/or cyst are relevant as these are often targets in procedures for removal or manipulation, and as such are interesting targets to track. As such, an organ model can be prepared for these as well, and adapted according to the method of the invention. As discussed further, these organ models are prepared separately, and are adapted separately based on the determined transformation (translation, rotation, resizing) of the tumor, cyst, fat tissue or other type of neoplasm, instead of being fixedly connected to another organ (model).

In a further preferred embodiment, when multiple separate organs are detected in the organ tissue in step d, steps e, f and g are performed separately for each of said detected separate organs.

This allows accurate tracking of each separate organ, as they are not rigidly attached to each other in terms of position and orientation, especially given the context of a medical procedure in which they are manipulated.

In a preferred embodiment, the step of automatically segmenting the digital images is performed for each pixel of the digital images, and classifies each of the pixel in one of the classes. By explicitly classifying each pixel as belonging to a certain category or class, any ambiguity is avoided with doubtful pixels, and allows to indicate the most likely classification explicitly.

In a preferred embodiment, the method comprises a step of providing the organ model adapted for a digital image as an organ model overlay over the digital image. By providing this as an overlay (potentially transparent or semi-transparent), the practitioner (or other viewers) can at all times keep a clear view on the position of the organ, even if it is partially occluded.

In a preferred embodiment, the method comprises a step of creating a non-organic overlay for the digital images, wherein the non-organic overlay only comprises the non-organic objects in the digital images, and wherein said non-organic overlay is applied over the organ model overlay. Most specifically, the step comprises a recognition of the non-organic object, for instance, from a certain library of tools and other (known and anticipated) objects (gauze, etc.) during a procedure, and an automatic 'completion' of the object based thereon, even if parts of the object is not visible in an image. Based on a partial recognition, the object, which in the case of tools is often rigid, can very easily be recognized and be estimated in terms of orientation, position and size, allowing such an automatic completion on limited visibility.

Generating separate overlays for the tools and objects and applying these on top of the original image allows the user to - at all times - see the tools and objects fully in the image, thus recognizing risks and danger, and allowing them to fully consider next steps and movements.

In some embodiments, this non-organic overlay can be partly transparent, allowing the interaction between tool and tissue to be even more clearly visible for the user. As mentioned, the above embodiment is especially relevant for tools which are manipulated by the practitioner (directly or via a mechanic/robotic construction).

In a preferred embodiment, the classes further comprise a class of non-organic objects, and the method comprises a step of detecting a tool based on the image segments identified as non-organic objects, and a step of determining interaction between a tool and the organ in one or more of the digital images based on the organ model in said one or more digital images and the non-organic object image segments in said one or more digital images.

In a preferred embodiment, the digital images comprise stereoscopic image data, and wherein the rotation or further rotation and/or resizing or further resizing and/or optional translation or further translation is determined for the subsequent or further subsequent digital image based on the stereoscopic image data of said subsequent or further subsequent digital image. Using the stereoscopic image data allows depth perception in the scene that is captured, and particularly of the organ tissue, which specifically allows the determination of the (further) rotation that has occurred in view of the preceding image.

Additionally, the use of two images allows artifact removal, corrections, and other quality-enhancing actions.

In a preferred embodiment, in case of insufficient organ tissue being present in a subsequent or further subsequent digital image, no organ model is adapted for said further or subsequent digital image, and no organ model is associated to said further or subsequent digital image.

As mentioned, preferably if there is no or insufficient organ tissue visible, the organ model is not 'updated', and no organ model will be associated to that image. In most cases, when the organ becomes visible (enough) again in later images, either step e is repeated, or the organ model adaption is based on the (timewise) closest preceding digital image with an associated organ model. Note that the second option can be further adapted by also factoring in similarity between the later image and a number of (timewise) close earlier images with an associated organ model, which can simplify the recognition of the transformation..

In a preferred embodiment, the preceding digital image and the subsequent further subsequent digital image are immediately subsequent digital images timewise.

In most cases, this will be most efficient, given that the change between the images will be minimal, thus allowing the transformation (rotation, translation, resizing) to be determined easily, and therefore quickly. Alternatively, the organ model is maintained over a number of subsequent images and only updated periodically (every X images) or dynamically (when a dissimilarity threshold is exceeded) to reduced computation load, especially at higher frame rates.

In a preferred embodiment, the preceding digital image and the subsequent further subsequent digital image are immediately subsequent digital images timewise, and in case of insufficient organ tissue being present in a subsequent or further subsequent digital image, no organ model is adapted for said further or subsequent digital image, and no organ model is associated to said further or subsequent digital image. If the preceding digital image does not have an associated organ model, step g for said further subsequent digital image is performed with an earlier digital image having an associated organ model serving as the preceding digital image, said earlier digital image being preceding to the preceding digital image, preferably said earlier digital image being the digital image having an associated organ model temporally closest to the further subsequent digital image.

In a preferred embodiment, the step of determining a rotation and/or resizing and/or optional translation is preceded by a step of identifying key features in the organ tissue of the organ in the subsequent digital image and the first digital image, said key features preferably comprising conjectured edges of the organ, and wherein the step of determining a rotation and/or resizing and/or optional translation is performed based on said identified key features in the subsequent digital image and the first digital image.

In a preferred embodiment, the step of determining a further rotation and/or resizing and/or optional translation is preceded by a step of identifying key features in the organ tissue of the organ in the further subsequent digital image and the preceding digital image, said key features preferably comprising conjectured edges of the organ, and wherein the step of determining a further rotation and/or resizing and/or optional translation is performed based on said identified key features in the further subsequent digital image and the preceding digital image.

In order to determine the transformation between two images, with the earlier having an established or adapted organ model, the later not, a transformation needs to be determined between the organ tissue of the organ between the two images. This is performed based on specific key features that are visually detectable in the images, and by mapping the change in position, size and orientation thereof onto each other, and aggregating this for all of these key features in the images. Specific key features can be edges of the organ tissue, areas of different coloration, areas with distinct demarcation, geometrically distinct features (such as connection structures, etc.), and others. If key features are visible in both of the two figures, then the transformation is derived based on the transformation of these shared key features.

In a further preferred embodiment, the preceding preferred embodiments regarding identifying key features and using these to determine the (further) rotation/resizing/optional translation, are combined.

In a preferred embodiment, the determination of the rotation and/or resizing and/or an optional translation is based on the subsequent digital image and the first digital image, and on a segmentation map of the subsequent digital image and the first digital image, wherein the segmentation map only comprises the image segments identified as organ tissue, and wherein the segmentation map comprises separate organ segmentation map for each of the detected organs separately.

In a preferred embodiment, the determination of the further rotation and/or resizing and/or an optional translation is based on the further subsequent digital image and the preceding digital image, and on a segmentation map of the further subsequent digital image and the preceding digital image, wherein the segmentation map only comprises the image segments identified as organ tissue, and wherein the segmentation map comprises separate organ segmentation map for each of the detected organs separately.

Using the segmentation map removes unnecessary (background, noise, etc.) information, allowing the transformation to be determined more efficiently. The segmentation map can be provided for one of the stereoscopic images, or for both each time.

In a further preferred embodiment, the preceding preferred embodiments regarding determining the (further) rotation/resizing/optional translation based on the digital images and the segmentation map for said digital images, are combined.

In a preferred embodiment, the digital images are preprocessed before the segmentation, the preprocessing potentially comprising the step of removing alpha-channel information from the images (which can be applied to the overlays), performing normalization of color channels (based on training set data for instance), resizing the image, cropping, etc.

In a preferred embodiment, the organ model is a deformable mesh, and step f further determines a deformation of the identified organ tissue of the organ in the subsequent digital image with respect to the identified organ tissue of the organ in the first digital image, and further adapts the three-dimensional organ for the subsequent digital image by transforming the organ model for the first image according to said determined deformation.

In a preferred embodiment, the organ model is a deformable mesh, and wherein step g further determines a deformation of the identified organ tissue of the organ in the further subsequent digital image with respect to the identified organ tissue of the organ in the preceding digital image, and further adapts the three-dimensional organ for the further subsequent digital image by transforming the organ model for the preceding digital image according to said determined deformation.

Using a deformable mesh allows the organ model to undergo deformation and thus more accurately reflect the organ in the images, preferably with some limiters being installed on the basis of established medical knowledge of the organ at hand (in order to comply with the laws of physics for the organ, and respecting internal structures and physical limiters, and not to simply provide for a general deformation limiter).

Given that organ in reality are deformable, and that during medical procedures, they are often manipulated by practitioners and/or tools, there is a need to also allow such deformations in the organ model that is applied to the images. If sufficient key features are determined for the organ tissue of an organ, it allows the transformation to be determined locally, providing a deformation mesh that can be applied to the organ model, and thereby allow deformation of the shape of the model.

Using a static (in shape) organ model simplifies the process computationally, but does not accurately reflect the reality. As such, given that the present invention substantially reduces the physical load, it allows deformations to be taken into account.

In a further preferred embodiment, the preceding preferred embodiments regarding the deformation of the organ mesh, are combined.

In a preferred embodiment, step e is performed by a pretrained machine learning model. Preferably, the machine learning model is a supervised machine learning model, or a semi-supervised machine learning model. In some embodiments, an unsupervised machine learning model can be applied.

In a preferred embodiment, the determination of the transformation (i.e., translation, rotation, resizing) in steps f and g is performed by a pretrained machine learning model.

Preferably, the machine learning model is a supervised learning-based transformation estimation model. The machine learning model is trained using a supervised learning approach with a dataset comprising pairs of medical images, where each pair includes a first image and a subsequent second image containing the same organ. The training dataset is annotated with ground truth transformations, including translation, rotation, and scaling. The model segments the organ in both images, determines a geometric transformation matrix, and outputs transformation parameters that map the organ in the first image to its corresponding position, orientation, and size in the second image.

Alternatively, the machine learning model is an unsupervised machine learning model. The model utilizes a feature extraction network to identify key anatomical landmarks on the organ in both the first and second medical images. The network then applies a transformation estimation module to compute an optimal transformation that aligns the organ in the second image with the corresponding organ in the first image. The transformation is iteratively refined using a loss function that minimizes the discrepancy between the aligned organs.

Alternatively, the machine learning model is a hybrid model, combining a convolutional neural network (CNN) with a traditional image registration algorithm. The CNN segments the organ in both the first and second medical images and extracts key features. A classical iterative closest point (ICP) algorithm is then applied to determine the optimal translation, rotation, and scaling parameters that align the organ from the first image to the second image.

Alternatively, the model is a transformer-based attention model for organ tracking, and is trained to analyze the spatial relationships between organ structures in a sequence of medical images. The model processes organ segmentations from the first and second images and uses an attention mechanism to predict the transformation matrix that best aligns the organ between the two images.

Alternatively, the model is a GAN (generative adversarial network) for organ matching, and comprises a generator network that predicts the transformation matrix for aligning the organ in the first image to the second image and a discriminator network that evaluates the accuracy of the predicted transformation. The system iteratively improves its predictions through adversarial training to generate more precise transformations.

Alternatively, the model is a reinforcement learning-based organ transformation prediction model, and is trained to predict transformations by iteratively applying small translation, rotation, and scaling adjustments to align the organ between the first and second images. The agent receives rewards based on the degree of overlap between the transformed organ and the actual segmented organ in the second image.

Alternatively, the model is a multi-scale segmentation and transformation estimation model, where organ structures are segmented at different resolutions. A hierarchical transformation model first aligns the organ at a coarse scale and then refines the transformation at finer scales, improving accuracy for small or complex anatomical structures.

Alternatively, the model is a deformable image registration model operating via Deep Learning. The model estimates a dense displacement field between the first and second segmented organ images, allowing for non-rigid transformation modeling. This method enables precise tracking of soft tissue deformations due to physiological movement or disease progression.

Alternatively, the model uses Bayesian inference for uncertainty-aware transformation estimation. A probabilistic Bayesian network is employed to estimate not only the transformation parameters but also the associated uncertainty. This is particularly useful for clinical applications where confidence in the transformation estimation affects subsequent decision-making.

The invention is further described by the following non-limiting examples which further illustrate the invention, and are not intended to, nor should they be interpreted to, limit the scope of the invention.

### EXAMPLES AND DESCRIPTION OF FIGURES

With as a goal illustrating better the properties of the invention the following presents, as an example and limiting in no way other potential applications, a description of a number of preferred applications of the method is provided below:

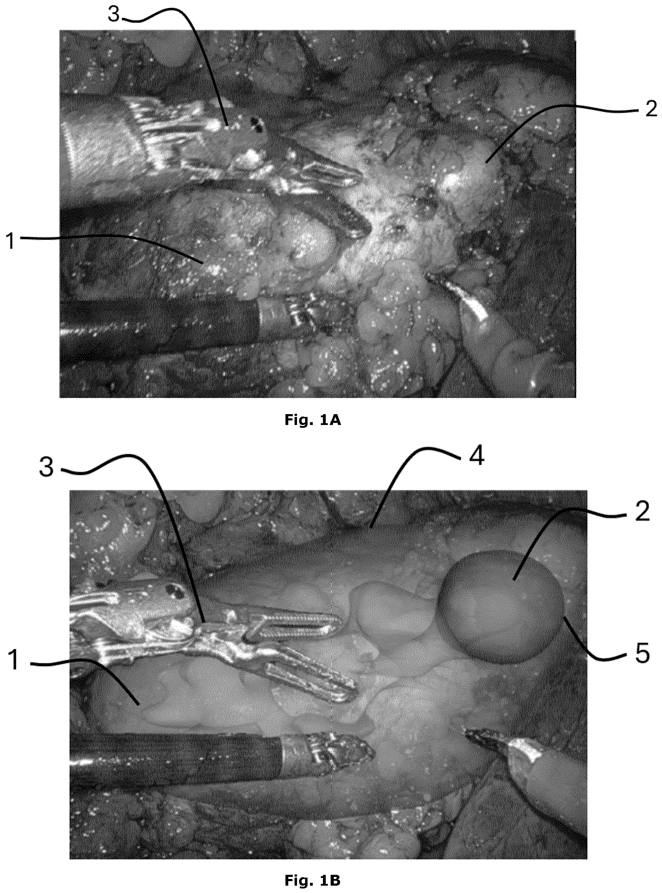

Figure 1A shows an unprocessed digital image during a medical procedure, in which 3 separate non-organic objects (3) are visible, along with an organ (1) on which a tumor (2) is present. In the method of the invention, the image is segmented, and the pixels are classified as organ tissue (1, 2) or other (3). Note that in the method of the invention, the tumor (2) and the organ (1) are considered as separate organs, and have separate organ models (4, 5).

In Figure 1B, a later digital image is shown that is provided with a separate organ model overlay (4, 5) for both the organ (1) and the tumor (2). This is the first image that is provided with the overlays (4, 5), and the first image having organ models associated thereto. These organ models are prepared based on the segmented original image, with the originally prepared organ models being mapped onto the image, based on the segmented image, such that the organ models (4, 5) substantially match the organ (1) and tumor (2). It is particularly noteworthy that the organ model overlays (4, 5) are provided on top of the medical image, excepting the non-organic objects, namely the tools (3), which are typically provided as a separate non-organic overlay on top of the image with the organ model overlays (4, 5).

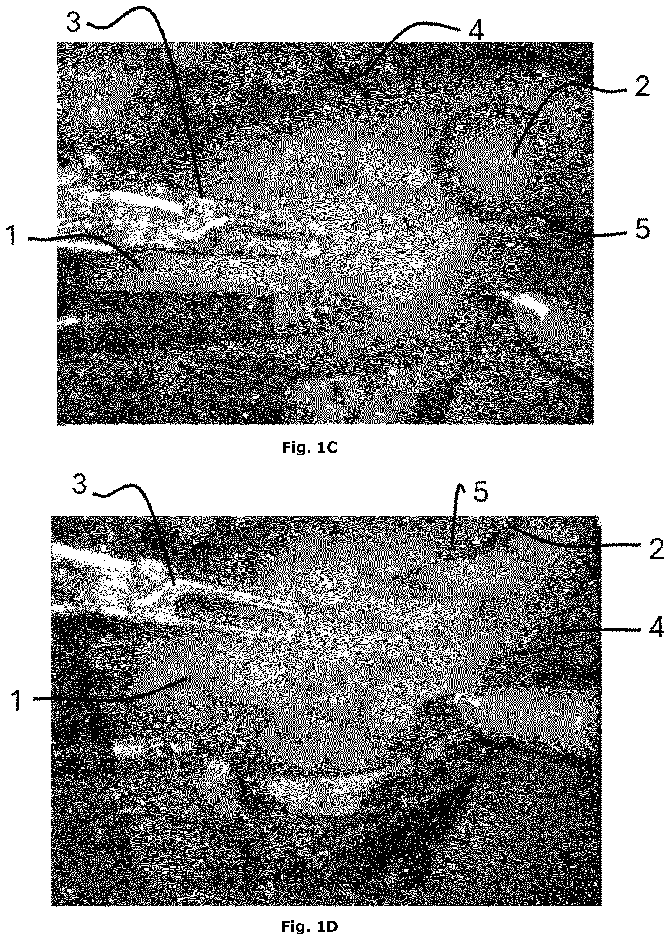

Figure 1C is taken at a later point in time, in which the organ (1) and tumor (2) have slightly shifted, as have the tools. The transformation of the organ (1) between the original digital image of Figure 1B (or another digital image preceding to that of Figure 1C) and the original digital image of Figure 1C is determined, in terms of rotation, translation and resizing. This transformation is then applied to the organ model associated to the digital image of Figure 1B (or as mentioned, the other digital image preceding to that of the Figure 1C), which transformed organ model is then associated to the digital image of Figure 1C as the organ model for said digital image, and can subsequently be used as the 'original' image and 'original' organ model for later images.

This leads to the transformed organ models (4, 5) for the organ (1) and the tumor (2), which are adapted separately, based on the separate transformations determined for the organ (1) and tumor (2) independently from each other.

Figure 1D shows an overlayed digital image at a further point in time, in which the differences are clearly visible, in terms of rotation, translation and resizing of the organ (1) and tumor (2) and the associated organ models (4, 5). Again, as mentioned, the tools (3) are provided on top of the image overlayed with the organ model overlays, as a further non-organic overlay.

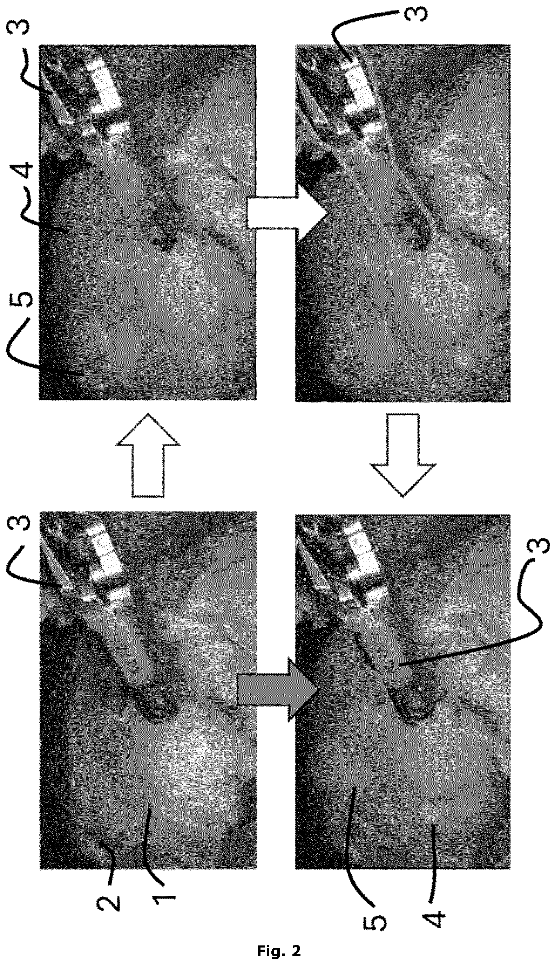

Figure 2 shows this latest point more clearly, indicating on the right figures where the tools are partially occluded by the organ model overlays (4, 5), which can result in uncertainty for the users. As a result, the present method envisions a further non-organic (tool) overlay (3) to be created, preferably again for each separate tool (3), which is overlayed as a (non-transparent) top layer over the digital image with the organ model overlays (4, 5).

It is supposed that the present invention is not restricted to any form of realization described previously and that some modifications can be added to the presented example without reappraisal of the appended claims. For example, the present invention has been described referring to organ detection primarily, but this can easily be applied to any type or collection of body tissue that is of relevance during a procedure (even though it is technically not an organ). Most specifically however, the invention applies to internal organs.

## Claims

1. Computer-implemented method for real-time organ detection in medical procedures on a patient, comprising the steps of:
a. receiving a plurality of digital images of a medical procedure, the plurality of digital images being a video;
b. automatically segmenting the digital images by a pretrained machine learning model into image segments and separating and identifying the segmented image segments in two or more classes, said classes comprising at least a class of organ tissue;
c. preparing one or more three-dimensional organ models based on historical image data of said patient;
d. based on the image segments identified as organ tissue, detecting at least one organ in a first digital image of the plurality of digital images;
e. adapting the prepared organ model of the detected organ for the first digital image, wherein the adaptation of said organ model is performed based on the identified organ tissue of the detected organ in said first digital image such that the organ model substantially corresponds to the detected organ in the first digital image in size and orientation, and optionally position;
**characterized in that** the method further comprises the steps of:
f. determining in at least one subsequent digital image to the first digital image, a rotation and/or resizing, and/or an optional translation of the identified organ tissue of the organ with respect to the identified organ tissue of the organ in the first digital image, and adapting the three-dimensional organ for the subsequent digital image by transforming the organ model for the first image according to said determined rotation and/or resizing and/or optional translation;
g. determining in at least one further subsequent digital image to the first digital image, preferably said further subsequent digital image being subsequent to the subsequent digital image, a further rotation and/or resizing and/or an optional translation of the identified organ tissue of the organ with respect to the identified organ tissue of the organ in a preceding digital image for which a three-dimensional organ model was adapted, and adapting the three-dimensional organ model for the further subsequent digital image by transforming the organ model for the preceding digital image according to said determined further rotation and/or resizing and/or optional translation.

2. Computer-implemented method according to the preceding claim 1, said classes further comprising a class of non-organic objects and a class of background tissue.

3. Computer-implemented method according any one of the preceding claims 1 or 2, wherein said class of organ tissue is subdivided in subclasses per organ.

4. Computer-implemented method according the preceding claim 3, wherein, when multiple separate organs are detected in the organ tissue in step d, steps e, f and g are performed separately for each of said detected separate organs.

5. Computer-implemented method according any one of the preceding claims 1 to 4, wherein the step of automatically segmenting the digital images is performed for each pixel of the digital images, and classifies each of the pixel in one of the classes.

6. Computer-implemented method according any one of the preceding claims 1 to 5, comprising a step of providing the organ model adapted for a digital image as an organ model overlay over the digital image.

7. Computer-implemented method according to claim 2 and 6, comprising a step of creating a non-organic overlay for the digital images, wherein the non-organic overlay only comprises the non-organic objects in the digital images, and wherein said non-organic overlay is applied over the organ model overlay.

8. Computer-implemented method according any one of the preceding claims 1 to 7, the classes further comprise a class of non-organic objects, the method comprising a step of detecting a tool based on the image segments identified as non-organic objects, and comprising a step of determining interaction between a tool and the organ in one or more of the digital images based on the organ model in said one or more digital images and the non-organic object image segments in said one or more digital images.

9. Computer-implemented method according any one of the preceding claims 1 to 8, wherein the digital images comprise stereoscopic image data, and wherein the rotation or further rotation and/or resizing or further resizing and/or optional translation or further translation is determined for the subsequent or further subsequent digital image based on the stereoscopic image data of said subsequent or further subsequent digital image.

10. Computer-implemented method according any one of the preceding claims 1 to 9, wherein in case of insufficient organ tissue being present in a subsequent or further subsequent digital image, no organ model is adapted for said further or subsequent digital image, and no organ model is associated to said further or subsequent digital image.

11. Computer-implemented method according any one of the preceding claims 1 to 10, wherein the preceding digital image and the subsequent further subsequent digital image are immediately subsequent digital images timewise.

12. Computer-implemented method according the preceding claims 10 and 11, wherein, if the preceding digital image does not have an associated organ model, step g for said further subsequent digital image is performed with an earlier digital image having an associated organ model serving as the preceding digital image, said earlier digital image being preceding to the preceding digital image, preferably said earlier digital image being the digital image having an associated organ model temporally closest to the further subsequent digital image.

13. Computer-implemented method according any one of the preceding claims 1 to 12, wherein the step of determining a rotation and/or resizing and/or optional translation or a further rotation and/or resizing and/or optional translation is preceded by a step of identifying key features in the organ tissue of the organ in the subsequent digital image and the first digital image or in the further subsequent digital image and the preceding digital image, said key features preferably comprising conjectured edges of the organ, and wherein the step of determining a rotation and/or resizing and/or optional translation or a further rotation and/or resizing and/or optional translation is performed based on said identified key features in the subsequent digital image and the first digital image or in the further subsequent digital image and the preceding digital image.

14. Computer-implemented method according any one of the preceding claims 1 to 13, wherein the determination of the rotation and/or resizing and/or an optional translation or determination of the further rotation and/or resizing and/or an optional translation is based on the subsequent digital image and the first digital image or on the further subsequent digital image and the preceding digital image, and on a segmentation map of the subsequent digital image and the first digital image or a segmentation map of the further subsequent digital image and the preceding digital image, wherein the segmentation map only comprises the image segments identified as organ tissue, and wherein the segmentation map comprises separate organ segmentation map for each of the detected organs separately.

15. Computer-implemented method according any one of the preceding claims 1 to 14, wherein the organ model is a deformable mesh, and wherein steps f and g respectively further determine a deformation of the identified organ tissue of the organ in the subsequent digital image with respect to the identified organ tissue of the organ in the first digital image or of the identified organ tissue of the organ in the further subsequent digital image with respect to the identified organ tissue of the organ in the preceding digital image, and respectively further adapt the three-dimensional organ for the subsequent digital image by transforming the organ model for the first image according to said determined deformation or further adapt the three-dimensional organ model for the further subsequent digital image by transforming the organ model for the preceding digital image according to said determined deformation.
